# NEW EUROPEAN PATENT SPECIFICATION

(11) **EP 2 546 224 B2**
(45) Date of publication and mention of the opposition decision: **14.06.2023**
(45) Mention of the grant of the patent: 26.09.2018
(21) Application number: 12178208.0
(22) Date of filing: 03.01.2007
(51) Int. Cl.: C07C 17/087, C07C 21/18

(54) **METHOD FOR PRODUCING FLUORINATED ORGANIC COMPOUNDS**
Verfahren zur Herstellung fluorinierter organischer Verbindungen
Procédé de production de composés organiques fluorés

(30) Priority: 03.01.2006 US 755485 P
(43) Date of publication of application: 16.01.2013
(62) Divisional of application: 07716229.5
(73) Proprietor: Honeywell International Inc., Morris Plains, NJ 07950 (US)
(72) Inventor: Merkel, Daniel, C., West Seneca, NY 14224 (US); Tung, Hsueh, Sung, Getzville, NY 14068 (US); Van der Puy, Michael, Amherst, NY 14221 (US); Ma, Jing, Ji, Morristown, NJ 07962-2245 (US); Dubey Rajesh, Buffalo, NY 14210 (US); Light, Barbara, Niagra Falls, NY 14305 (US); Bortz, Cheryl, N Tonawanda, NY 14120 (US); Phillips, Steven, D., Buffalo, NY 14218 (US); Mukhopahyay, Sudip, Morristown NJ 07962-2245 (US)
(74) Representative: Crooks, Elizabeth Caroline

(56) References cited:
- EP-A1- 0 939 071
- EP-B1- 2 546 225
- WO-A1-98/42645
- WO-A2-2005/012212
- FR-A1- 2 748 473
- US-A- 2 437 993
- US-A- 2 670 387
- US-A- 4 900 874
- HENNE, ALBERT L. ET AL: "Fluorinated derivatives of propane and propylene. VI", JOURNAL OF THE AMERICAN CHEMICAL SOCIETY, vol. 68, 1946, pages 496-497, XP002448570,
- PALETA, OLDRICH ET AL: "Synthesis of perfluoroallyl chloride and some chlorofluoropropenes", BULLETIN DE LA SOCIETE CHIMIQUE DE FRANCE, no. 6, 1986, pages 920-924, XP009088473,
- MARIA O. BURGIN ET AL: "UNIMOLECULAR REACTION KINETICS OF CF2CLCF2CH3 AND CF2CLCF2CD3: EXPERIMENTAL EVIDENCE FOR A NOVEL 1,2-FCL REARRANGEMENT PATHWAY", J. PHYS. CHEM. A, vol. 105, 2001, pages 1615-1621, XP002448571,
- R. N. HASZELDINE: "550. Reactions of fluorocarbon radicals. Part V. Alternative syntheses for trifluoromethylacetylene (3 : 3 : 3-trifluoropropyne), and the influence of polyfluoro-groups on adjacent hydrogen and halogen atoms", JOURNAL OF THE CHEMICAL SOCIETY (RESUMED), 1 January 1951 (1951-01-01), page 2495, XP55056602, ISSN: 0368-1769, DOI: 10.1039/jr9510002495
- WILLIAM T MILLER ET AL: "SUBSTITUTION AND ADDITION REACTIONS OF THE FLUOROOLEFINS. IV. REACTIONS OF FLUORIDE ION WITH FLUOROOLEFINS", JOURNAL OF THE AMERICAN CHEMICAL SOCIETY, ACS PUBLICATIONS, US, vol. 82, 20 June 1960 (1960-06-20), pages 3091-3099, XP002326736, ISSN: 0002-7863, DOI: 10.1021/JA01497A028
- ALBERT L. HENNE ET AL: JOURNAL OF THE AMERICAN CHEMICAL SOCIETY, vol. 67, no. 10, 19 October 1945 (1945-10-19), pages 1639-1640, XP55056613, ISSN: 0002-7863, DOI: 10.1021/ja01226a002
- MILLER: "Chapter 32: Preparation of Fluorocarbons by Polymerization of Olefins", 1 January 1951 (1951-01-01), NATION. NUCLEAR ENERGY SER. ABT,, PAGE(S) 567 - 685, XP009168043, * under point 14: 7th and 8th line; page 666 * * under point 19: 7th and 8th line; page 667 * * under point 21: 5th line; page 667 *
- E. T. MCBEE ET AL: "Fluorinated Derivatives of Propane", JOURNAL OF THE AMERICAN CHEMICAL SOCIETY, vol. 69, no. 4, 19 April 1947 (1947-04-19) , pages 944-947, XP055044256, ISSN: 0002-7863, DOI: 10.1021/ja01196a065
- HEARD et al.: "1,2-FCl Rearrangement as an Intermediate Step in the Unimolecular 1,3-HCl Elimination from Chlorofluoropropanes", J. Phys. Chem. A, vol. 105, March 2001 (2001-03), pages 1622-1625, XP055416203,
- Ullmann's Encyclopedia of Industrial Chemistry, vol. 15 (2012), p. 443-494
- Feiring, A E., Journal of Fluorine Chemistry, vol. 13 (1979), p. 7-18
- Zhu, L. et al , J. Phys. Chem. A, vol 110(2006), p. 1506-1517
- Jones, G., Journal of Chemical Education, vol. 38, no. 6 (1961), p. 297-300
- Xing Qiyi et al., Basic Organic Chemistry, Higher Education Press, 3rd edition (2005)

## Description

### BACKGROUND OF INVENTION

### (1) Field of Invention:

This invention relates to novel methods for preparing fluorinated organic compounds.

### (2) Description of Related Art:

Hydrofluorocarbons (HFCs), in particular hydrofluoroalkenes such tetrafluoropropenes (including 2,3,3,3-tetrafluoro-1-propene (HFO-1234yf) and 1,3,3,3-tetrafluoro-1-propene (HFO-1234ze) have been disclosed to be effective refrigerants, fire extinguishants, heat transfer media, propellants, foaming agents, blowing agents, gaseous dielectrics, sterilant carriers, polymerization media, particulate removal fluids, carrier fluids, buffing abrasive agents, displacement drying agents and power cycle working fluids. Unlike chlorofluorocarbons (CFCs) and hydrochlorofluorocarbons (HCFCs), both of which potentially damage the Earth's ozone layer, HFCs do not contain chlorine and thus pose no threat to the ozone layer.

Several methods of preparing hydrofluoroalkenes are known. For example, U.S. Pat. No. 4,900,874 (Ihara et al) describes a method of making fluorine containing olefins by contacting hydrogen gas with fluorinated alcohols. Although this appears to be a relatively high-yield process, for commercial scale production the handling of hydrogen gas at high temperature raises difficult safety related questions. Also, the cost of producing hydrogen gas, such as building an on-site hydrogen plant, can be in many situations prohibitive.

U.S. Pat. No. 2,931,840 (Marquis) describes a method of making fluorine containing olefins by pyrolysis of methyl chloride and tetrafluoroethylene or chlorodifluoromethane. This process is a relatively low yield process and a very large percentage of the organic starting material is converted in this process to unwanted and/or unimportant byproducts.

WO 98/42645 discloses the preparation of 2-chloro-2,3,3,3-tetrafluoropropane from fluoromethane and chlorotrifluoroethylene.

J. Am. Chem. Soc., Vol. 69, no. 4, 1947, pages 944-947 discloses the preparation of 2-chloro-2,3,3,3-tetrafluoropropane by fluorination of CF₃CCl₂CH₃.

The preparation of HFO-1234yf from trifluoroacetylacetone and sulfur tetrafluoride has been described. *See* Banks, et al., Journal of Fluorine Chemistry, Vol. 82, Iss. 2, p. 171-174 (1997). Also, U.S. Pat. No. 5,162,594 (Krespan) discloses a process wherein tetrafluoroethylene is reacted with another fluorinated ethylene in the liquid phase to produce a polyfluoroolefin product.

### SUMMARY

The present invention relates to a method for producing a compound of formula (II) comprising producing a compound of formula (IB)

CF₃CClFCH₃

by fluorinating a compound of formula (IAA)

CH₂=CClCF₃

to produce a compound of formula (IB),
wherein the reaction is carried out in the liquid or gas phase; and
dehydrohalogenating said compound of formula (IB) to form a compound of Formula (II),

   CF₃CF=CH₂ (II).

As used herein and throughout, unless specifically indicated otherwise, the term "converting" includes directly converting (for example, in a single reaction or under essentially one set of reaction conditions, an example of which is described hereinafter) and indirectly converting (for example, through two or more reactions or using more than a single set of reaction conditions).

### DETAILED DESCRIPTION OF PREFERRED EMBODIMENTS

### FLUORINATION OF THE COMPOUND OF FORMULA (IAA)

The compound of Formula (IAA), is subject to fluorination reaction(s) to produce a compound of Formula (IB) (HCFC-244bb). Preferably this gas phase reaction is at least partially catalyzed.

The fluorination of the compound of Formula (IAA) is preferably carried out under conditions effective to provide a Formula (IAA) conversion of at least about 40%, more preferably at least about 50%, and even more preferably at least about 60%. Further in certain preferred embodiments, the conversion of the compound of Formula (IAA) comprises reacting such compound under conditions effective to produce HCFC-244bb, at a selectivity of at least about 70%, more preferably at least about 80%, and even more preferably at least about 85%, with selectivities of about 90% or greater being achieved in certain embodiments.

In general, it is possible that this fluorination reaction step can be carried out in the liquid phase or in the gas phase, or in a combination of gas and liquid phases, and it is contemplated that the reaction can be carried out batch wise, continuous, or a combination of these.

For embodiments in which the reaction comprises a liquid phase reaction, the reaction can be catalytic or non-catalytic. Preferably, a catalytic process is used. Lewis acid catalyst, such as metal-halide catalysts, including antimony halides, tin halides, thallium halides, iron halides, and combinations of two or more of these, are preferred in certain embodiments. Metal chlorides and metal fluorides are particularly preferred. Examples of particularly preferred catalysts of this type include SbCl₅, SbCl₃, SbF₅, SnCl₄, TiCl₄, FeCl₃ and combinations of two or more of these.

In preferred gas phase fluorination of a Formula (IAA) compound, the reaction is at least partially a catalyzed reaction, and is preferably carried out on a continuous basis by introducing a stream containing the compound of Formula (IAA) into one or more reaction vessels, such as a tubular reactor. In certain preferred embodiments, the stream containing the compound of Formula (IAA), is preheated to a temperature of from about 50°C to about 400°C, and in certain embodiments preferably about 80°C. In other embodiments, it is preferred that the stream containing the compound of Formula (IAA), is preheated to a temperature of from about 150°C to about 400°C, preferably about 300°C. This stream, preferably after preheating, is then preferably introduced into a reaction vessel (preferably a tube reactor), which is maintained at the desired temperature, preferably from about 50°C to about 250°C, more preferably from about 50°C to about 150°C, where it is preferably contacted with catalyst and fluorinating agent, such as HF.

Preferably the vessel is comprised of materials which are resistant to corrosion such as Hastelloy, Inconel, Monel and/or fluoropolymers linings.

Preferably the vessel contains catalyst, for example a fixed or fluid catalyst bed, packed with a suitable fluorination catalyst, with suitable means to ensure that the reaction mixture is maintained within about the desired reaction temperature range.

Thus, it is contemplated that the fluorination reaction step may be performed using a wide variety of process parameters and process conditions in view of the overall teachings contained herein. However, it is preferred in certain embodiments that this reaction step comprise a gas phase reaction, preferably in the presence of catalyst, and even more preferably an Sb-based catalyst, such as catalyst which is 50 wt% SbCl₅/C. Other catalysts which may be used include: from 3 to 6 wt% FeCl₃/C; SbF₅/C; 20 wt% SnCl₄/C; 23 wt% TiCl₄/C; and activated carbon. Preferably the catalyst comprises Cl₂ and HF pre-treated SbCl₅/C.

In general it is also contemplated that a wide variety of reaction pressures may be used for the fluorination reaction, depending again on relevant factors such as the specific catalyst being used and the most desired reaction product. The reaction pressure can be, for example, superatmospheric, atmospheric or under vacuum and in certain preferred embodiments is from 7 to 1379 kPa (1 to 200 psia), more preferably in certain embodiments from 7 to 827 kPa (1 to 120 psia).

In certain embodiments, an inert diluent gas, such as nitrogen, may be used in combination with the other reactor feed(s).

It is contemplated that the amount of catalyst use will vary depending on the particular parameters present in each embodiment.

### DEHYDROHALOGENATION OF FORMULA (IB)

The compound of Formula (IB) (HCFC-244bb) is dehydrohalogenated to produce a compound of Formula (II),

CF₃CF=CH₂ (II).

In certain preferred embodiments, the stream containing the compound of Formula (IB) is preheated to a temperature of from about 150°C to about 400°C, preferably about 350°C, and introduced into a reaction vessel, which is maintained at about the desired temperature, preferably from about 200°C to about 700°C, more preferably from about 300°C to about 700°C, more preferably from about 300°C to about 450°C, and more preferably in certain embodiments from about 350°C to about 450°C.

Preferably the vessel is comprised of materials which are resistant to corrosion as Hastelloy, Inconel, Monel and/or fluoropolymers linings. Preferably the vessel contains catalyst, for example a fixed or fluid catalyst bed, packed with a suitable dehydrohalogenation catalyst, with suitable means to heat the reaction mixture to about the desired reaction temperature.

Thus, it is contemplated that the dehydrohalogenation reaction step may be performed using a wide variety of process parameters and process conditions in view of the overall teachings contained herein. However, it is preferred in certain embodiments that this reaction step comprise a gas phase reaction, preferably in the presence of catalyst, and even more preferably a carbon- and/or metal-based catalyst, preferably activated carbon, a nickel-based catalyst (such as Ni-mesh) and combinations of these. Other catalysts and catalyst supports may be used, including palladium on carbon, palladium-based catalyst (including palladium on aluminum oxides), and it is expected that many other catalysts may be used depending on the requirements of particular embodiments in view of the teachings contained herein. Of course, two or more any of these catalysts, or other catalysts not named here, may be used in combination.

The gas phase dehydrohalogenation reaction may be conducted, for example, by introducing a gaseous form of a compound of Formula (IB) into a suitable reaction vessel or reactor. Preferably the vessel is comprised of materials which are resistant to corrosion as Hastelloy, Inconel, Monel and/or fluoropolymers linings. Preferably the vessel contains catalyst, for example a fixed or fluid catalyst bed, packed with a suitable dehydrohalogenation catalyst, with suitable means to heat the reaction mixture to about the desired reaction temperature.

While it is contemplated that a wide variety of reaction temperatures may be used, depending on relevant factors such as the catalyst being used and the most desired reaction product, it is generally preferred that the reaction temperature for the dehydrohalogenation step is from about 200°C to about 800°C, more preferably from about 400°C to about 800°C, and even more preferably from about 400°C to about 500°C, and more preferably in certain embodiments from about 300°C to about 500°C.

In general it is also contemplated that a wide variety of reaction pressures may be used, depending again on relevant factors such as the specific catalyst being used and the most desired reaction product. The reaction pressure can be, for example, superatmospheric, atmospheric or under vacuum, and in certain preferred embodiments is from about 7 to about 1379 kPa (about 1 to about 200 psia), and even more preferably in certain embodiments from about 7 to about 827 kPa (about 1 to about 120 psia).

In certain embodiments, an inert diluent gas, such as nitrogen, may be used in combination with the other reactor feed(s). When such a diluent is used, it is generally preferred that the compound of Formula (IB), comprise from about 50% to greater than 99% by weight based on the combined weight of diluent and Formula (IB) compound.

It is contemplated that the amount of catalyst use will vary depending on the particular parameters present in each embodiment.

Preferably in such dehydrofluorination embodiments as described in this section, the conversion of the Formula (IB) compound is at least about 60%, more preferably at least about 75%, and even more preferably at least about 90%. Preferably in such embodiments, the selectivity to compound of Formula (II), HFO-1234yf, is at least about 50%, more preferably at least about 70% and more preferably at least about 80%.

### EXAMPLES

Additional features of the present invention are provided in the following examples.

### Example 1 (Reference Example)

### Selective catalyzed-transformation of CCl₂=CClCH₂Cl to CF₃CCl=CH₂ (HFO-1233xf) in gas-phase

A 56 cm (22-inch) long and 1.27 cm (1/2-inch) diameter Monel pipe gas-phase reactor is charged with about 120 cc of a catalyst or a mixture of two catalysts. In case of a mixture, Cr₂O₃ catalyst is kept at the bottom zone of the reactor at a constant temperature of about 270°C-500°C and the other catalyst, such as FeCl₃/C, is kept at the middle and the top zone of the reactor at a constant temperature of about 120°C - 220°C. The reactor is mounted inside a heater with three zones (top, middle, and bottom). The reactor temperature is read by custom-made-5-point thermocouples kept inside at the middle of the reactor. The bottom of the reactor is connected to a pre-heater, which is kept at 300°C by electrical heating. The liquid-HF is fed from a cylinder into the pre-heater through a needle valve, liquid mass-flow meter, and a research control valve at a constant flow of about 1 to about 1000 grams pre hour (g/h). The HF cylinder is kept at a constant pressure of 412 kPa (45 psig) by applying anhydrous N₂ gas pressure into the cylinder head space. About 10 to about 1000 g/h of CCl₂=CClCH₂Cl is fed as a liquid through a dip tube from a cylinder under about 412 kPa (45 psig) of N₂ pressure. The organic flows from the dip tube to the preheater (kept at about 250°C) through a needle valve, liquid mass-flow meter, and a research control valve at a constant flow of 1-1000 g/h. The organic is also fed as a gas while heating the cylinder containing organic at about 220°C. The gas coming out of the cylinder is passed through a needle valve and a mass flow controller into the preheater. The organic line from the cylinder to the pre-heater is kept at about 200°C by wrapping with constant temperature heat trace and electrical heating elements. All feed cylinders are mounted on scales to monitor their weight by difference. The catalysts are dried at the reaction temperature over a period of about 8 hours and then pretreated with about 50 g/h of HF under atmospheric pressure over a period of about 6 hours and then under 446 kPa (50 psig) HF pressure over another period of about 6 hours before contacting with organic feed containing CCl₂=CClCH₂Cl. The reactions are run at a constant reactor pressure of about 101 to 1136 kPa (0 to about 150 psig) by controlling the flow of reactor exit gases by another research control valve. The gases exiting reactor are analyzed by on-line GC and GC/MS connected through a hotbox valve arrangement to prevent condensation. The conversion of CCl₂=CClCH₂Cl is about 70 to about 100% and the selectivity to 1233xf is about 80% to about 95%, respectively. The product is collected by flowing the reactor exit gases through a scrubber solution comprising about 20 wt% to about 60 wt% KOH in water and then trapping the exit gases from the scrubber into a cylinder kept in dry ice or liquid N₂. The product, 1233xf is then substantially isolated by distillation. The results are tabulated in Table 1.

**Table 1: Transformation of CCl₂=CClCH₂Cl to CF₃CCl=CH₂ (CCl₂=CClCH₂Cl + 3HF → CF₃CCl=CH₂ + 3HCl)**

| **#** | **Catalyst** | **T, °C** | **HF flow, g/h** | **CCl**₂**=CClCH₂Cl flow, g/h** | **% Conv of CCl₂=CClCH₂Cl** | **% Sel to 1233xf** |
|---|---|---|---|---|---|---|
| **1** | 10% v/v Cr₂O₃-90% v/v FeCl₃/C | 350/150 | 50 | 12 | 79 | 81 |
| **2** | 20% v/v Cr₂O₃-80% v/v FeCl₃/C | 350/150 | 50 | 12 | 83 | 86 |
| **3** | 30% v/v Cr₂O₃-70% v/v FeCl₃/C | 350/150 | 50 | 12 | 89 | 96 |
| **4** | 30% v/v Cr₂O₃-70% v/v FeCl₃/C | 350/150 | 70 | 12 | 79 | 93 |
| **5** | 30% v/v Cr₂O₃-70% v/v FeCl₃/C | 345/170 | 50 | 25 | 85 | 90 |
| **6** | Cr₂O₃ | 350 | 50 | 20 | 90 | 93 |
| **7** | FeCl₃/C | 150 | 50 | 20 | 74 | 39 |
| **8** | SbCl₅/C | 150 | 50 | 20 | 81 | 52 |
| Reaction conditions: Catalyst used (total) 120 cc; pressure, 122 kPa (1.5 psig); | | | | | | |

### Examples 2A and 2B

### Liquid-phase catalytic fluorination of CF₃CCl=CH₂ (1233xf) with HF to CF₃CFClCH₃ (244bb)

### Example 2A

About 327 grams of HF, about 50 grams 1233xf, and about 75 grams SbCl₅ were charged into a 1-L autoclave. The reaction mixture was stirred at a temperature of about 80°C for about 3 hours under about 4376 kPa (620 psig) of pressure. After the reaction, the reactor was cooled to about 0°C and about 300 ml water was then added slowly into the autoclave over a period of about 45 min. After complete addition of water under stirring, the reactor was cooled to room temperature and then the overhead gases were transferred to another collecting cylinder. The yield of CF₃CFClCH₃ was about 90% at a 1233xf conversion level of about 98%. The other major by-products were CF₃CF₂CH₃ (2%), and an unidentified isomer of a C4 compound of the general formula, C₄H₃Cl₃F₄ (8%).

### Example 2B

About 327 grams HF, about 50 grams 1233xf, and about 75 grams SbCl₅ were charged into a 1-L autoclave. The reaction mixture was stirred at 80°C for about 3 hours under about 4411 kPa (625 psig) of pressure. After the reaction, the reactor was cooled to about 45°C and then the overhead gas mixture was passed through a well dried KF, NaF, or Al₂O₃ (350 g) packed column kept at about 80°C to strip off HF from the gas stream. The gases coming out of the column are collected in a cylinder kept in dry ice (-70°C) bath. The yield of CF₃CFClCH₃ was 87% at a 1233xf conversion level of 93%. The other major by-products were CF₃CF₂CH₃ (1%), and an unidentified isomer of a C4 compound of the general formula, C₄H₃Cl₃F₄ (7%). The product, CF₃CFClCH₃ was isolated by distillation with 98% purity.

### Example 3

### Gas-phase catalytic fluorination of CF₃CCl=CH₂ (1233xf) with HF to CF₃CFClCH₃ (244bb)

A 56 cm (22-inch) 1.27 cm (1/2-inch) diameter Monel tube gas phase reactor was charged with about 120 cc of a catalyst. The reactor was mounted inside a heater with three zones (top, middle and bottom). The reactor temperature was read by a custom made 5-point thermocouple kept at the middle inside of the reactor. The inlet of the reactor was connected to a pre-heater, which was kept at about 300°C by electrical heating. Organic (1233xf) was fed from a cylinder kept at 70°C through a regulator, needle valve, and a gas mass-flow-meter. The organic line to the pre-heater was heat traced and kept at a constant temperature of about 73°C by electrical heating to avoid condensation. N₂ was used as a diluent in some cases and fed from a cylinder through a regulator and a mass flow controller into the pre-heater. All feed cylinders were mounted on scales to monitor their weight by difference. The reactions were run at a constant reactor pressure of from about 101 to 791 kPa (0 to about 100 psig) by controlling the flow of reactor exit gases by another research control valve. The gas mixtures exiting reactor was analyzed by on-line GC and GC/MS connected through a hotbox valve arrangements to prevent condensation. The conversion of 1233xf was from about 50% to about 65% and the selectivity to 244bb isomer (CF₃CFClCH₃) was from about 90% to about 93% depending on the reaction conditions using 120 cc of 50 wt% SbCl₅/C as the catalyst at about 65°C to about -85°C with a HF flow of about 50 g/h and organic flow of about 15 g/h. No CF₃CF₂CH₃ was observed under the reaction conditions. The catalyst is pretreated at first with 50 g/h HF at about 65°C for about 2 hours and then with about 50 g/h HF and about 200 sccm of Cl₂ at about 65°C for about 4 hours. After pre-treatment, about 50 sccm of N₂ is flows over a period of about 40 minutes through the catalyst bed to sweep free chlorine from the catalyst surface prior to interacting with the organic feed (1233xf). Pretreatment is considered important to many embodiments of the invention. The products were collected by flowing the reactor exit gases through a 20-60 wt% aqueous KOH scrubber solution and then trapping the exit gases from the scrubber into a cylinder kept in dry ice or liquid N₂. The products were then isolated by distillation. About 50 wt% SbCl₅/C, about 3 to about 6 wt% FeCl₃/C, 20 wt% SnCl₄/C, and about 23 wt% TiCl₄/C, using 4 different kind of activated carbon such as Shiro saga, Calgon, Norit, and Aldrich were used as the catalyst at from about 60 to about 150°C. Among all the catalysts used for this reaction, Cl₂ and HF pre-treated SbCl₅/C was found to be generally preferred in terms of activity. The results using SbCl₅ as the catalyst are shown in Table 2.

**Table 2: Catalyzed-gas-phase transformation of CF₃CCl=CH₂ to CF₃CFClCH₃**

| # | Cat | T, °C | Conv. of CF₃CCl=CH₂ (1233xf) | Sel. to CF₃CFClCH₃ |
|---|---|---|---|---|
| 1 | 10 wt% SbCl₅/C | 60 | 15 | 100 |
| 2 | 20 wt% SbCl₅/C | 60 | 21 | 98 |
| 3 | 30 wt% SbCl₅/C | 60 | 32 | 98 |
| 4 | 50 wt% SbCl₅/C | 60 | 55 | 97 |
| 5 | 50 wt% SbCl₅/C | 80 | 62 | 93 |
| 6 | 50 wt% SbCl₅/C | 100 | 56 | 87 |
| 7 | 60 wt% SbCl₅/C | 60 | 59 | 91 |
| 8 | 50 wt% SbCl₅/NORIT RFC 3 Activated Carbon | 60 | 34 | 92 |
| 9 | 50 wt% SbCl₅/Shiro Saga Activated Carbon | 60 | 56 | 96 |
| 10 | 50 wt% SbCl₅/Aldrich Activated Carbon | 60 | 57 | 94 |
| **Reaction conditions:** 1233xf flow, 150 sccm; HF flow 50 g/h; pressure, 119 to 138 kPa (2.5-5.3 psig); in 1-5 reactions Calgon activated carbon is used as the catalyst support; catalyst, 120 cc. All catalysts are pre-treated with Cl₂ and HF prior to contacting with 1233xf. | | | | |

### Example 4

### Conversion of CF₃CFClCH₃ to CF₃CF=CH₂ in gas-phase

A 56 cm (22-inch) 1.27 cm (1/2-inch) diameter Monel tube gas phase reactor was charged with 120 cc of catalyst. The reactor was mounted inside a heater with three zones (top, middle and bottom). The reactor temperature was read by custom made 5-point thermocouples kept at the middle inside of the reactor. The inlet of the reactor was connected to a pre-heater, which was kept at about 300°C by electrical heating. Organic (CF₃CFClCH₃) was fed from a cylinder kept at about 65°C through a regulator, needle valve, and a gas mass-flow-meter. The organic line to the pre-heater was heat traced and kept at a constant temperature of from about 65°C to about 70°C by electrical heating to avoid condensation. The feed cylinder was mounted on scales to monitor their weight by difference. The reactions were run at a constant reactor pressure of from about 101 to 791 kPa (0 to about 100 psig) by controlling the flow of reactor exit gases by another research control valve. The gas mixture exiting reactor was analyzed by on-line GC and GC/MS connected through a hotbox valve arrangement to prevent condensation. The conversion of CF₃CFClCH₃ was almost 98% and the selectivity to HFO-1234yf was from about 69% to about 86% depending on the reaction conditions. The products were collected by flowing the reactor exit gases through a about 20 wt% to about 60 wt% of aqueous KOH scrubber solution and then trapping the exit gases from the scrubber into a cylinder kept in dry ice or liquid N₂. The products were then isolated by distillation. Results are tabulated in Table 3.

**Table 3: Catalyzed-transformation of CF₃CFClCH₃ to HFO-1234yf**

| # | Cat | T, °C | | Flow rate, CF₃CFClCH₃ (244bb isomer) sccm | | Conversion of 244bb | | 1234yf (Sel. %) | |
|---|---|---|---|---|---|---|---|---|---|
| 1 | A | 400 | | | 150 | | 100 | | 46 |
| 2 | B | 400 | | | 150 | | 96 | | 63 |
| 3 | C | 400 | | | 100 | | 100 | | 64 |
| 4 | D | 400 | | | 100 | | 99 | | 93 |
| 5 | D | 400 | | | 150 | | 92 | | 89 |
| 6 | E | 400 | | | 100 | | 96 | | 56 |
| 7 | F | 400 | | | 100 | | 87 | | 51 |
| 8 | G | 400 | | | 100 | | 100 | | 37 |
| **Reaction conditions:** pressure, 119 to 138 kPa (2.5-5.3 psig); catalyst, 100 cc, A is NORIT RFC 3; B is Shiro-Saga activated carbon; C is Aldrich activated carbon; D is Calgon activated carbon; activated carbon; E is 0.5 wt% Pd/C; F is 0.5 wt% Pt/C; G is Ni-mesh; Organic cylinder temperature-65°C; CF₃CFClCH₃ (244bb) line to the preheater-60°C; Preheater, 350°C; P-136 kPa (5 psig). | | | | | | | | | |

### Example 5 (Reference Example)

### Selective catalyzed-transformation of CCl₃CCl=CH₂ to CF₃CCl=CH₂ (HFO-1233xf) in gas-phase

A 56 cm (22-inch) long and 1.27 cm (1/2-inch) diameter Monel pipe gas phase reactor was charged with 120 cc of a catalyst or a mixture of two catalysts. In case of a mixture, Cr₂O₃ catalyst is kept at the bottom zone of the reactor at a substantially constant temperature of from about 270°C to about 500°C and the other catalyst, such as FeCl₃/C is kept at the middle and the top zone of the reactor at a substantially constant temperature of from about 120°C to about 220°C. The reactor was mounted inside a heater with three zones (top, middle, and bottom). The reactor temperature was read by custom-made-5-point thermocouples kept inside at the middle of the reactor. The bottom of the reactor was connected to a pre-heater, which was kept at about 300°C by electrical heating. The liquid-HF was fed from a cylinder into the pre-heater through a needle valve, liquid mass-flow meter, and a research control valve at a substantially constant flow of from about 1 to about 1000 g/h. The HF cylinder was kept at a substantially constant pressure of about 412 kPa (45 psig) by applying anhydrous N₂ gas pressure into the cylinder head space. A feed rate of from about 10 g/h to about 1000 g/h of CCl₃CCl=CH₂ was fed as a liquid through a dip tube from a cylinder under about 412 kPa (45 psig) of N₂ pressure. The organic was flown from the dip tube to the pre-heater (kept at about 250°C) through needle valve, liquid mass-flow meter, and a research control valve at a substantially constant flow of from about 1 to about 1000 g/h. The organic is also fed as a gas while heating the cylinder containing organic at about 220°C. The gas effluent from the cylinder is passed through a needle valve and a mass flow controller into the pre-heater. The organic line from the cylinder to the pre-heater was kept at about 200°C by wrapping with constant temperature heat trace and electrical heating elements. All feed cylinders were mounted on scales to monitor their weight by difference. The catalysts were dried at the reaction temperature over a period of about 8 hours and then pretreated with about 50 g/h of HF under atmospheric pressure over a 6 hour period and then under about 446 kPa (50 psig) HF pressure over a 6 hour period before contacting with organic feed, CCl₃CCl=CH₂. The reactions were run at a substantially constant reactor pressure ranging from about 101 to 1136 kPa (0 to about 150 psig) by controlling the flow of reactor exit gases by another research control valve. Those gases exiting reactor were analyzed by on-line GC and GC/MS connected through a hotbox valve arrangements to prevent condensation. The conversion of CCl₃CCl=CH₂ was in a range of from about 90% to about 100% and the selectivity to CF₃CCl=CH₂ (1233xf) was about 79%. The effluent contained in addition HFO-1243zf in an amount of about 7.7%, 1232-isomer in an amount of about 1.3%, and 1223 in an amount of about 0.8%, and an unidentified byproduct. The product was collected by flowing the reactor exit gases through a 20-60 wt% aq. KOH scrubber solution and then trapping the exit gases from the scrubber into a cylinder kept in dry ice or liquid N₂. The product, 1233xf was then substantially isolated by distillation. Using only Cr₂O₃ catalyst, a selectivity of about 68% to 1233xf at a conversion level of about 79% was achieved.

## Claims

1. A method for producing a compound of formula (II) comprising producing a compound of formula (IB)
CF₃CClFCH₃
by fluorinating a compound of formula (IAA)
CH₂=CClCF₃
to produce a compound of formula (IB),
wherein the reaction is carried out in the liquid or gas phase; and
dehydrohalogenating said compound of formula (IB) to form a compound of Formula (II),
CF₃CF=CH₂ (II).

2. The method of claim 1, wherein when said reaction to produce said compound of formula (IB) is carried out in the liquid phase, said reaction is catalysed by a metal-halide.

3. The method of claim 2 wherein said catalyst is antimony halide, tin halide, thallium halide, iron halide or combinations of two or more of these.

4. The method of claim 3 wherein said catalyst is SbCl₅.

5. The method of claim 1 wherein when said reaction to produce said compound of formula (IB) is carried out in the gas phase, said reaction is catalysed by a Sb-based catalyst.

6. The method of claim 1 wherein said reaction to produce said compound of formula (IB) is carried out in the gas phase, and said catalyst is 50 wt% SbCl₅/C, 3-6 wt% FeCl₃/C, SbFs/C, 20 wt% SnCl₄/C, 23 wt% TiCl₄/C or activated carbon.

7. The method of claim 1 wherein said reaction to produce said compound of formula (IB) is carried out in the gas phase, and said catalyst is 50 wt% SbCl₅/C.

8. The method of any one of claims 1 to 7, wherein dehydrohalogenating said compound of Formula (IB) to form a compound of Formula (II) comprises a gas phase reaction.

9. The method of claim 8, wherein the gas phase reaction is in the presence of a catalyst.

## Patentansprüche

1. Verfahren zur Herstellung einer Verbindung der Formel (II), umfassend das Herstellen einer Verbindung der Formel (IB)
CF₃CClFCH₃
durch Fluorieren einer Verbindung der Formel (IAA)
CH₂=CClCF₃
um eine Verbindung der Formel (IB) herzustellen,
wobei die Reaktion in der flüssigen oder Gasphase ausgeführt wird; und
Dehydrohalogenieren der Verbindung der Formel (IB), um eine Verbindung der Formel (II) zu bilden,
CF₃CF=CH₂ (II).

2. Verfahren nach Anspruch 1, wobei, wenn die Reaktion zur Herstellung der Verbindung der Formel (IB) in der flüssigen Phase ausgeführt wird, die Reaktion durch ein Metallhalogenid katalysiert wird.

3. Verfahren nach Anspruch 2, wobei der Katalysator Antimonhalogenid, Zinnhalogenid, Thalliumhalogenid, Eisenhalogenid oder Kombinationen von zwei oder mehr dieser ist.

4. Verfahren nach Anspruch 3, wobei der Katalysator SbCl₅ ist.

5. Verfahren nach Anspruch 1, wobei, wenn die Reaktion zur Herstellung der Verbindung der Formel (IB) in der Gasphase ausgeführt wird, die Reaktion durch einen Katalysator auf der Basis von Sb katalysiert wird.

6. Verfahren nach Anspruch 1, wobei die Reaktion zur Herstellung der Verbindung der Formel (IB) in der Gasphase ausgeführt wird und der Katalysator 50 Gew.-% SbCl₅/C, 3-6 Gew.-% FeCl₃/C, SbF₅/C, 20 Gew.-% SnCl₄/C, 23 Gew.-% TiCl₄/C oder Aktivkohle ist.

7. Verfahren nach Anspruch 1, wobei die Reaktion zur Herstellung der Verbindung der Formel (IB) in der Gasphase ausgeführt wird und der Katalysator 50 Gew.-% SbCl₅/C ist.

8. Verfahren nach einem der Ansprüche 1 bis 7, wobei das Dehydrohalogenieren der Verbindung der Formel (IB), um eine Verbindung der Formel (II) herzustellen, eine Gasphasenreaktion umfasst.

9. Verfahren nach Anspruch 8, wobei die Gasphasenreaktion in Gegenwart eines Katalysators ausgeführt wird.

## Revendications

1. Procédé de production d'un composé de formule (II) comprenant la production d'un composé de formule (IB)
CF₃CClFCH₃
par fluoration d'un composé de formule (IAA)
CH₂=CClCF₃
pour produire un composé de formule (IB),
dans lequel la réaction est conduite en phase liquide ou gazeuse ; et
déshydrohalogénation dudit composé de formule (IB) pour former un composé de formule (II),
CF₃CF=CH₂ (II).

2. Procédé selon la revendication 1, dans lequel lorsque ladite réaction pour produire ledit composé de formule (IB) est conduite en phase liquide, ladite réaction est catalysée par un halogénure métallique.

3. Procédé selon la revendication 2, dans lequel ledit catalyseur est un halogénure d'antimoine, un halogénure d'étain, un halogénure de thallium, un halogénure de fer ou des combinaisons de deux d'entre eux ou plus.

4. Procédé selon la revendication 3, dans lequel ledit catalyseur est SbCl₅.

5. Procédé selon la revendication 1, dans lequel lorsque ladite réaction pour produire ledit composé de formule (IB) est conduite en phase gazeuse, ladite réaction est catalysée par un catalyseur à base de Sb.

6. Procédé selon la revendication 1, dans lequel ladite réaction pour produire ledit composé de formule (IB) est mise en œuvre en phase gazeuse, et ledit catalyseur est 50 % en poids de SbCl₅/C, 3 à 6 % en poids de FeCl₃/C, SbF₅/C, 20 % en poids de SnCl₄/C, 23 % en poids de TiCl₄/C ou du charbon actif.

7. Procédé selon la revendication 1, dans lequel ladite réaction pour produire ledit composé de formule (IB) est mise en œuvre en phase gazeuse, et ledit catalyseur est 50 % en poids de SbCl₅/C.

8. Procédé selon l'une quelconque des revendications 1 à 7, dans lequel la déshydrohalogénation dudit composé de formule (IB) pour former un composé de formule (II) comprend une réaction en phase gazeuse.

9. Procédé selon la revendication 8, dans lequel la réaction en phase gazeuse est conduite en présence d'un catalyseur.
